# EUROPEAN PATENT APPLICATION

(11) **EP 4 332 102 A1**
(43) Date of publication of application: **06.03.2024**
(21) Application number: 22795014.4
(22) Date of filing: 29.04.2022
(51) Int. Cl.: C07D 471/04, A61K 31/519, A61P 11/06, A61P 11/08, A61P 37/08

(54) **ISOQUINOLONE COMPOUND AND USE THEREOF**

(30) Priority: 29.04.2021 CN 202110472916; 28.05.2021 CN 202110594411
(71) Applicant: Suzhou Suncadia Biopharmaceuticals Co., Ltd., Suzhou, Jiangsu 215126 (CN); Jiangsu Hengrui Pharmaceuticals Co., Ltd., Lianyungang, Jiangsu 222047 (CN)
(72) Inventor: LI, Zhiya, Suzhou, Jiangsu 215126 (CN); WU, Jie, Suzhou, Jiangsu 215126 (CN); HU, Yimin, Suzhou, Jiangsu 215126 (CN); WANG, Guobao, Suzhou, Jiangsu 215126 (CN); YANG, Changyong, Lianyungang, Jiangsu 222047 (CN); HUANG, Xiaoxing, Lianyungang, Jiangsu 222047 (CN); ZHANG, Jinyi, Lianyungang, Jiangsu 222047 (CN)
(74) Representative: Potter Clarkson
(86) International application number: PCT/CN2022/090175
(87) International publication number: WO 2022/228544

(57) **Abstract**

The present invention relates to an isoquinolone compound and a use thereof. Specifically, the present invention provides a 4-H pyrimido[6,1-a]isoquinolin-4-one compound represented by formula I or a pharmaceutically acceptable salt thereof, or a stereoisomer, rotamer, or tautomer thereof, wherein R¹, R², E¹, E², ring Cy, m, and n are defined as the present text.

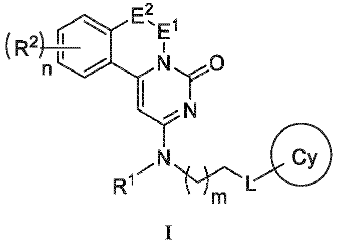

## Description

### TECHNICAL FIELD

The disclosure belongs to the field of pharmaceutics, and relates to isoquinolinone compounds and use thereof.

### BACKGROUND

Phosphodiesterases (PDEs) belong to a superfamily of enzyme systems including 11 families, each of which plays a role in a different signal transduction and regulates a different physiological process. Among them, PDE3 has the ability to hydrolyze both cAMP and cGMP, and the ability for cAMP is about ten times greater than that for cGMP. There are two genetic subtypes of PDE3: PDE3A and PDE3B, which are located on chromosome 11 and chromosome 12, respectively. According to start codon, PDE3A can be subdivided into three subtypes: PDE3A1, PDE3A2 and PDE3A3, which are present mainly in the heart, platelets, the vascular smooth muscle, and oocytes, serving to regulate myocardial contractility, platelet aggregation, vascular smooth muscle contraction, oocyte maturation, renin release, etc. There is only one subtype of PDE3B: PDE3B1, which is mainly present in lipocytes, liver cells, spermatocytes and the pancreas and is mainly involved in the regulation of the signal transduction of insulin, insulin-like growth factors and leptin. It plays an important role in metabolic diseases such as obesity and diabetes. Main PDE3 selective inhibitors include cilostazol, cilostamide, milrinone, amrinone, enoximone, siguazodan, etc.

For example, amrinone inhibits PDE3 activity, increases the cAMP concentration in myocardial cells and increases the Ca²⁺ concentration in cells, thereby producing a positive inotropic effect to the full. Meanwhile, amrinone directly acts on vascular smooth muscle cells, producing a good vasodilating effect, increasing myocardial contractility, reducing pulmonary artery pressure and restoring heart and lung function. It has great value in the treatment of chronic pulmonary heart disease combined with heart failure. In addition, cilostazol is clinically used for treating anti-platelet aggregation, pulmonary hypertension (PAH), chronic obstructive pulmonary disease (COPD), intermittent claudication, and cerebral small vessel disease.

In another aspect, PDE4 is highly specific for cAMP, and there are 4 subtypes of PDE4: PDE4A, PDE4B, PDE4C and PDE4D. PDE4 is involved in the promotion of monocyte and macrophage activation, neutrophil infiltration, vascular smooth muscle proliferation, vasodilatation, myocardial contraction and other related pathophysiological processes, and has effects on central nervous system function, cardiovascular function, inflammation/the immune system, cell adhesion, etc. PDE4 plays a leading regulatory role in the expression of pro- and anti-inflammatory mediators. PDE4 inhibitors can inhibit the release of harmful mediators from inflammatory cells.

A new molecule with both PDE3 and PDE4 inhibitory activities can cause bronchodilation like a β-adrenergic receptor agonist and anti-inflammatory action like an inhaled glucocorticoid. The two complementary targeting abilities can achieve a better effect than a single one.

For example, RPL554 (9,10-dimethoxy-2-(2,4,6-trimethylphenylimino)-3-(N-carbamoyl-2-aminoethyl)-3,4,6, 7-tetrahydro-2H-pyrimido[6,l-a]isoquinolin-4-one) is a PDE3/PDE4 dual-target inhibitor disclosed in WO00/58308A1. Recent phase II clinical data show that the drug can significantly improve bronchodilation and symptoms in patients with chronic obstructive pulmonary disease, and that the drug was well tolerated and did not cause noticeable adverse events; for example, problems with the heart, nausea and diarrhea were all mild. The safety of the drug and its "limited systemic exposure" are encouraging.

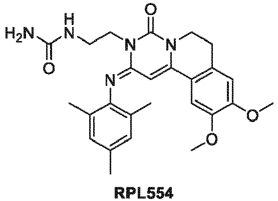

In addition, other companies have also been active in the development of PDE3/4 molecules; for example, published related patent applications include WO2016040083, WO2020011254, WO2018020249, WO2014140647, WO2020011254, etc. However, to better meet market demand, there is still a need to develop new, high-efficiency low-toxicity selective PDE inhibitors.

### SUMMARY

The disclosure provides a compound of formula I or a pharmaceutically acceptable salt thereof, 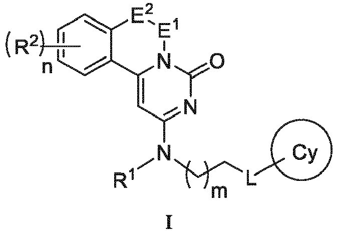
wherein R¹ is aryl or heteroaryl, and the aryl or heteroaryl is optionally substituted with one or more R^{A1};
each R^{A1} is independently selected from the group consisting of deuterium, halogen, hydroxy, nitro, amino, cyano, C₁₋₆ alkyl, C₁₋₆ alkoxy, C₃₋₇ cycloalkyl, C₃₋₇ cycloalkoxy, 3- to 7-membered heterocycloalkyl, 3- to 7-membered heterocycloalkoxy, C₆₋₁₀ aryl and 5- to 10-membered heteroaryl, and the alkyl, alkoxy, cycloalkyl, cycloalkoxy, heterocycloalkyl, heterocycloalkoxy, aryl and heteroaryl are each independently optionally substituted with one or more substituents selected from the group consisting of deuterium, halogen, hydroxy, oxo, nitro, cyano and amino;
each R² is independently selected from the group consisting of deuterium, halogen, hydroxy, nitro, amino, cyano, C₁₋₆ alkyl, C₂₋₇ alkenyl, C₃₋₇ cycloalkyl, 3- to 7-membered heterocycloalkyl, C₁₋₆ alkoxy, C₂₋₇ alkenyloxy, C₃₋₇ cycloalkoxy, 3- to 7-membered heterocycloalkoxy, C₆₋₁₀ aryl and 5- to 10-membered heteroaryl, and the hydroxy, amino, alkyl, alkenyl, cycloalkyl, heterocycloalkyl, alkoxy, alkenyloxy, cycloalkoxy, heterocycloalkoxy, aryl and heteroaryl are optionally substituted with one or more R^{A2};
each R^{A2} is independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, cycloalkoxy, heterocycloalkoxy, aryl and heteroaryl are each independently optionally substituted with one or more substituents selected from the group consisting of deuterium, halogen, hydroxy, oxo, nitro, cyano and amino;
E¹ is -(CH₂)_{q}-;
q is selected from the group consisting of 1, 2, 3 and 4;
E² is selected from the group consisting of -O-, -NH-, -S- and a single bond;
m is selected from the group consisting of 0, 1, 2, 3 and 4;
n is selected from the group consisting of 0, 1, 2, 3 and 4;
L is selected from the group consisting of -N(R⁶)-, -N(R⁶)C(O)-, -C(O)N(R⁶)-, -S-, -OC(O)-, -C(O)O-, -C(O)N(R⁶)CH₂-, -N(R⁶)C(O)CH₂-, -NHS(O)₂-, -S(O)₂NH- and a single bond;
R⁶ is selected from the group consisting of hydrogen, hydroxy and C₁₋₃ alkyl, and the alkyl is optionally substituted with one or more substituents selected from the group consisting of deuterium, halogen, hydroxy, oxo, nitro, amino and cyano;
ring Cy is heterocycloalkyl or heteroaryl, and the heterocycloalkyl or heteroaryl is optionally substituted with one or more R^{A3},
each R^{A3} is independently selected from the group consisting of deuterium, halogen, hydroxy, nitro, cyano, C₁₋₆ alkyl, C₃₋₇ cycloalkyl, 3- to 7-membered heterocycloalkyl, 6-to 10-membered aryl, 5- to 10-membered heteroaryl, SR¹, SOR', SO₂R', SO₂NR'(R"), NR'(R"), COOR' and CONR'(R"), and the alkyl, cycloalkyl, heterocycloalkyl, aryl, and heteroaryl are each independently optionally substituted with one or more substituents selected from the group consisting of deuterium, halogen, hydroxy, oxo, nitro, cyano and amino; and
R' and R" are each independently selected from the group consisting of hydrogen, deuterium, hydroxy, C₁₋₆ alkyl, C₃₋₇ cycloalkyl, 3- to 7-membered heterocycloalkyl, C₆₋₁₀ aryl and 5- to 10-membered heteroaryl, and the alkyl, cycloalkyl, heterocycloalkyl, aryl and heteroaryl are each independently optionally substituted with one or more substituents selected from the group consisting of deuterium, halogen, hydroxy, oxo, nitro, cyano and amino.

In some embodiments, in the compound of formula I, E¹ is -(CH₂)q-, and q is 1 or 2.

In some embodiments, in the compound of formula I, E² is a single bond.

In some embodiments, in the compound of formula I, E¹ is -(CH₂)q-, and q is 1 or 2; and E² is a single bond.

In some embodiments, in the compound of formula I, E² is -NH-.

In some embodiments, in the compound of formula I, E¹ is -(CH₂)q-, and q is 1 or 2; and E² is -NH-.

In some other embodiments, in the compound of formula I, m is 1 or 2.

In some embodiments, in the compound of formula I, E¹ is -(CH₂)q-, and q is 1 or 2; E² is -NH-; and m is 1 or 2.

In some embodiments, in the compound of formula I, E¹ is -(CH₂)q-, and q is 1 or 2; E² is a single bond; and m is 1 or 2.

In some embodiments, in the compound of formula I, ring Cy is 5- to 6-membered heterocycloalkyl, and the heterocycloalkyl is optionally substituted with one or more R^{A3}, and R^{A3} is as defined above.

Further, in some embodiments, in the compound of formula I, ring Cy is selected from the group consisting of 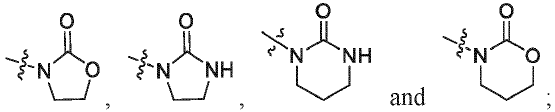 further, the ring Cy is optionally substituted with one or more R^{A3}, and R^{A3} is as defined above.

In another aspect, in the compound of formula I provided by some embodiments, ring Cy is 5- to 10-membered heteroaryl, and the heteroaryl is optionally substituted with one or more R^{A3}, and R^{A3} is as defined above. In some embodiments, in the compound of formula I, ring Cy is selected from the group consisting of 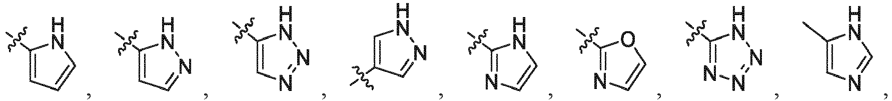 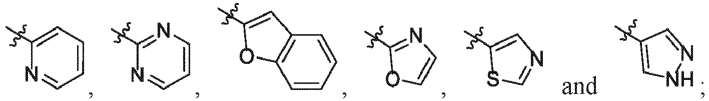 further, the ring Cy is optionally substituted with one or more R^{A3}, and R^{A3} is as defined above.

In some other embodiments, in the compound of formula I, ring Cy is 9- to 10-membered heteroaryl, and the heteroaryl is optionally substituted with one or more R^{A3}, and R^{A3} is as defined above. The ring Cy is selected from the group consisting of 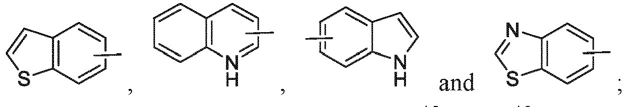 further, the ring Cy is optionally substituted with one or more R^{A3}, and R^{A} is as defined above.

In some embodiments, in the compound of formula I, each R^{A3} is independently selected from the group consisting of deuterium, halogen, hydroxy, amino and C₁₋₆ alkyl, and the alkyl is optionally substituted with one or more substituents selected from the group consisting of deuterium, halogen, hydroxy, oxo, nitro, cyano and amino.

In some other embodiments, in the compound of formula I, each R^{A3} is independently selected from the group consisting of deuterium, halogen, hydroxy, amino, C₃₋₇ cycloalkyl and 3- to 7-membered heterocycloalkyl, and the cycloalkyl or heterocycloalkyl is optionally substituted with one or more substituents selected from the group consisting of deuterium, halogen, hydroxy, oxo, nitro, cyano and amino.

In another aspect, in the compound of formula I provided by some embodiments, R¹ is C₆₋₁₀ aryl, and the aryl is optionally substituted with one or more R^{A1}, and R^{A1} is as defined above. Further, the compound of formula I is a compound of formula II,

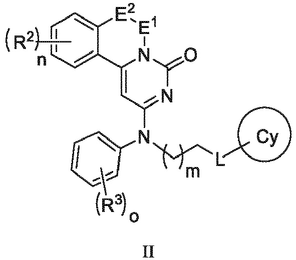

In the compound of formula II provided by some embodiments, each R³ is independently selected from the group consisting of deuterium, halogen, hydroxy, amino, cyano, C₁₋₆ alkyl and C₁₋₆ alkoxy, and the alkyl is optionally substituted with one or more substituents selected from the group consisting of deuterium, halogen, hydroxy, oxo, nitro, cyano and amino. In the compound of formula II provided by some other embodiments, each R³ is independently selected from the group consisting of C₃₋₇ cycloalkyl, C₃₋₇ cycloalkoxy, 3- to 7-membered heterocycloalkyl and 3- to 7-membered heterocycloalkoxy, and the cycloalkyl, cycloalkoxy, heterocycloalkyl and heterocycloalkoxy are each independently optionally substituted with one or more substituents selected from the group consisting of deuterium, halogen, hydroxy, oxo, nitro, cyano and amino. In the compound of formula II provided by some other embodiments, each R³ is independently C₆₋₁₀ aryl or 5- to 10-membered heteroaryl, and the aryl or heteroaryl is optionally substituted with one or more substituents of deuterium, halogen, hydroxy, oxo, nitro, cyano and amino.

Further, in the compound of formula I or formula II provided by some embodiments, each R² is independently selected from the group consisting of deuterium, halogen, hydroxy, amino and cyano.

In some other embodiments, in the compound of formula I or formula II, each R² is independently selected from the group consisting of C₁₋₆ alkoxy, C₂₋₇ alkenyloxy, C₃₋₇ cycloalkoxy and 3- to 7-membered heterocycloalkoxy, and the alkoxy, alkenyloxy, cycloalkoxy and heterocycloalkoxy are optionally substituted with one or more R^{A2}, and R^{A2} is as defined above.

In some other embodiments, in the compound of formula I or formula II, n is selected from the group consisting of 0, 1 and 3. In some other embodiments, in the compound of formula I or formula II, n is 2.

In some embodiments, in the compound of formula II, R³ is C₁₋₆ alkyl, such as methyl, ethyl or propyl.

In some embodiments, in the compound of formula II, R³ is C₁₋₆ alkoxy, such as methoxy, ethoxy or propoxy.

In some embodiments, in the compound of formula I, R¹ is 2,4,6-trimethylphenyl or 2,4,6-trimethoxyphenyl.

In another aspect, in the compound of formula I or formula II, L is selected from the group consisting of -N(R⁶)-, -N(R⁶)C(O)-, -C(O)N(R⁶)-, -NHS(O)₂- and a single bond.

In some embodiments, in the compound of formula I or formula II, L is -N(R⁶)-. In some embodiments, in the compound of formula I or formula II, L is -N(R⁶)C(O)-. In some embodiments, in the compound of formula I or formula II, L is -C(O)N(R⁶)-. Further, in certain embodiments, in the compound of formula I or formula II, R⁶ is hydrogen or C₁₋₃ alkyl (e.g., methyl, ethyl or propyl), and the alkyl is optionally substituted with one or more substituents selected from the group consisting of deuterium, halogen, hydroxy, oxo, nitro, amino and cyano. In certain embodiments, in the compound of formula I or formula II, R⁶ is hydrogen. In certain embodiments, in the compound of formula I or formula II, R⁶ is methyl.

In some other embodiments, in the compound of formula I or formula II, L is

-NHS(O)₂-. In some embodiments, in the compound of formula I or formula II, L is a single bond.

In another aspect, the compound of formula I or formula II is a compound of formula III, 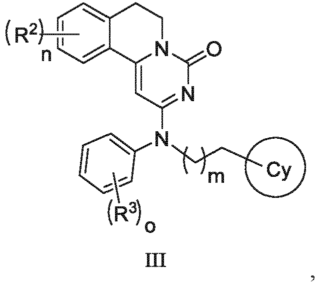 wherein ring Cy, R², m, n, R³ and o are as defined above.

In some embodiments, in the compound of formula I or formula II or formula III, ring Cy is 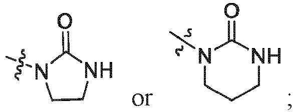 further, the ring Cy is optionally substituted with one or more R^{A3}, and R^{A3} is as defined above.

In certain embodiments, the compound of formula I is a compound of formula IV, 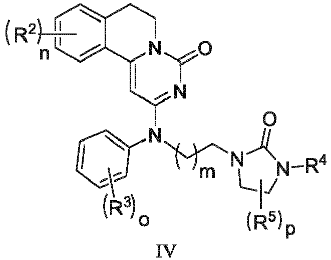
wherein R⁴ is selected from the group consisting of hydrogen, C₁₋₆ alkyl and C₃₋₇ cycloalkyl, and the alkyl and cycloalkyl are each independently optionally substituted with one or more substituents selected from the group consisting of deuterium, halogen, hydroxy, oxo, nitro, cyano and amino;
each R⁵ is independently selected from the group consisting of deuterium, halogen, hydroxy, nitro, cyano, C₁₋₆ alkyl, C₃₋₇ cycloalkyl, 3- to 7-membered heterocycloalkyl, 6-to 10-membered aryl and 5- to 10-membered heteroaryl, and the alkyl, cycloalkyl, heterocycloalkyl, aryl and heteroaryl are each independently optionally substituted with one or more substituents selected from the group consisting of deuterium, halogen, hydroxy, oxo, nitro, cyano and amino;
p is selected from the group consisting of 0, 1, 2, 3 and 4;
R², m and n are as defined in the compound of formula I, and
R³ and o are as defined in the compound of formula II.

In some embodiments, in the compound of formula IV, R⁴ is hydrogen or C₁₋₆ alkyl (e.g., C₁₋₃ alkyl, methyl, ethyl and propyl), and the alkyl is optionally substituted with one or more substituents selected from the group consisting of halogen, hydroxy and amino.

In some other embodiments, in the compound of formula IV, each R⁵ is independently selected from the group consisting of deuterium, halogen, hydroxy, nitro and cyano.

In some other embodiments, in the compound of formula IV, each R⁵ is independently selected from the group consisting of C₁₋₆ alkyl, C₃₋₇ cycloalkyl and 3- to 7-membered heterocycloalkyl, and the alkyl, cycloalkyl and heterocycloalkyl are each independently optionally substituted with one or more substituents selected from the group consisting of deuterium, halogen, hydroxy, oxo, nitro, cyano and amino.

In some other embodiments, in the compound of formula IV, each R⁵ is independently 6- to 10-membered aryl (e.g., phenyl) or 5- to 10-membered heteroaryl (e.g., 5- to 6-membered heteroaryl, pyridine; and 9- to 10-membered heteroaryl), and the aryl or heteroaryl is optionally substituted with one or more substituents selected from the group consisting of deuterium, halogen, hydroxy, oxo, nitro, cyano and amino. Further, the compound of formula I is a compound of formula V, 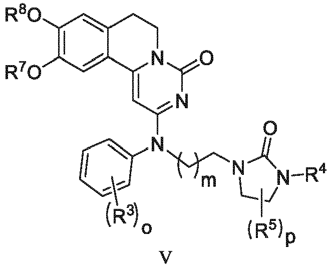
wherein R⁷ is selected from the group consisting of hydrogen, deuterium, C₁₋₆ alkyl, C₂₋₇ alkenyl, C₃₋₇ cycloalkyl and 3- to 7-membered heterocycloalkyl, and the alkyl, alkenyl, cycloalkyl and heterocycloalkyl are each independently optionally substituted with one or more R^{A4};
R^{A4} is selected from the group consisting of deuterium, halogen, hydroxy, oxo, nitro, amino, cyano, C₁₋₆ alkyl, C₁₋₆ alkoxy, C₃₋₇ cycloalkyl, 3- to 7-membered heterocycloalkyl, C₆₋₁₀ aryl and 5- to 10-membered heteroaryl, and the alkyl, alkoxy, cycloalkyl and heterocycloalkyl are each independently optionally substituted with one or more substituents selected from the group consisting of deuterium, halogen, hydroxy, oxo, nitro, cyano and amino;
R⁸ is selected from the group consisting of hydrogen, deuterium, C₁₋₆ alkyl, C₂₋₇ alkenyl, C₃₋₇ cycloalkyl and 3- to 7-membered heterocycloalkyl, and the alkyl, alkenyl, cycloalkyl and heterocycloalkyl are each independently optionally substituted with one or more R^{A5};
R^{A5} is selected from the group consisting of deuterium, halogen, hydroxy, oxo, nitro, amino, cyano, C₁₋₆ alkyl, C₁₋₆ alkoxy, C₃₋₇ cycloalkyl, 3- to 7-membered heterocycloalkyl, C₆₋₁₀ aryl and 5- to 10-membered heteroaryl, and the alkyl, alkoxy, cycloalkyl and heterocycloalkyl are each independently optionally substituted with one or more substituents of deuterium, halogen, hydroxy, oxo, nitro, cyano and amino;
m is as defined in the compound of formula I;
R³ and o are as defined in the compound of formula II; and
R⁴, R⁵ and p are as defined in the compound of formula V.

In some embodiments, in the compound of formula V, R⁸ is hydrogen or C₁₋₆ alkyl, and the alkyl is optionally substituted with one or more R^{A5}, and R^{A5} is as defined above.

In some embodiments, in the compound of formula V, R⁸ is C₂₋₇ alkenyl (e.g., C₂₋₄ alkenyl, propenyl or allyl) or 3- to 7-membered heterocycloalkyl (e.g., 3- to 5-membered heterocycloalkyl), and the alkenyl or heterocycloalkyl is optionally substituted with one or more R^{A5}, and R^{A5} is as defined above.

In some embodiments, in the compound of formula V, R⁸ is C₃₋₇ cycloalkyl, and the cycloalkyl is optionally substituted with one or more R^{A5}, and R^{A5} is as defined above. In some embodiments, in the compound of formula V, R^{A5} is selected from the group consisting of deuterium, halogen, hydroxy, oxo, nitro, amino and cyano.

In some embodiments, in the compound of formula V, R^{A5} is selected from the group consisting of C₃₋₇ cycloalkyl, 3- to 7-membered heterocycloalkyl and C₆₋₁₀ aryl, and the cycloalkyl or heterocycloalkyl is optionally substituted with one or more substituents selected from the group consisting of deuterium, halogen, hydroxy, oxo, nitro and cyano.

In some embodiments, in the compound of formula V, R^{A5} is selected from the group consisting of C₁₋₆ alkyl, C₁₋₆ alkoxy and 5- to 10-membered heteroaryl, and the alkyl, alkoxy and heteroaryl are each independently optionally substituted with one or more substituents selected from the group consisting of deuterium, halogen, hydroxy, oxo, nitro and cyano.

In another aspect, in some embodiments, in the compound of formula V, R⁷ is selected from the group consisting of hydrogen, C₁₋₆ alkyl and C₃₋₇ cycloalkyl, and the alkyl and cycloalkyl are each independently optionally substituted with one or more R^{A4}, and R^{A4} is as defined above.

In some embodiments, in the compound of formula V, R⁷ is C₂₋₇ alkenyl or 3- to 7-membered heterocycloalkyl, and the alkenyl or heterocycloalkyl is optionally substituted with one or more R^{A4}, and R^{A4} is as defined above.

In some other embodiments, in the compound of formula V, R^{A4} is selected from the group consisting of deuterium, halogen, hydroxy, oxo, nitro, amino and cyano.

In some other embodiments, in the compound of formula V, R^{A4} is selected from the group consisting of C₁₋₆ alkyl, C₁₋₆ alkoxy, C₃₋₇ cycloalkyl, 3- to 7-membered heterocycloalkyl, C₆₋₁₀ aryl and 5- to 10-membered heteroaryl, and the alkyl, alkoxy, cycloalkyl and heterocycloalkyl are each independently optionally substituted with one or more substituents of deuterium, halogen, hydroxy, oxo, nitro and cyano.

In another aspect, in the compound of formula I or the pharmaceutically acceptable salt thereof provided by some embodiments, R¹ is selected from the group consisting of 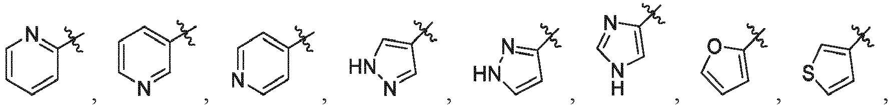 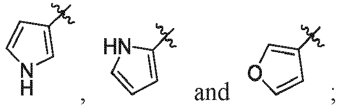 further, R¹ is optionally substituted with one or more RA1.

In some embodiments, in the compound of formula I or the pharmaceutically acceptable salt thereof, R¹ is selected from the group consisting of 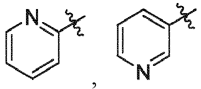 and 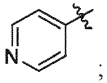 further, R¹ is optionally substituted with one or more R^{A1}.

In some embodiments, in the compound of formula I or the pharmaceutically acceptable salt thereof, R¹ is selected from the group consisting of 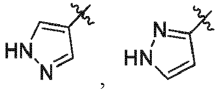 and 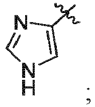 further, R¹ is optionally substituted with one or more R^{A1}.

In some embodiments, in the compound of formula I or the pharmaceutically acceptable salt thereof, R¹ is selected from the group consisting of 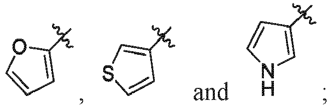 further, R¹ is optionally substituted with one or more R^{A1}.

In another aspect, in some embodiments, in the compound of formula I or the pharmaceutically acceptable salt thereof, ring Cy is 7- to 9-membered heterocycloalkyl, and the heterocycloalkyl is optionally substituted with one or more R^{A3}. In certain embodiments, in the compound of formula I or the pharmaceutically acceptable salt thereof, ring Cy is selected from the group consisting of 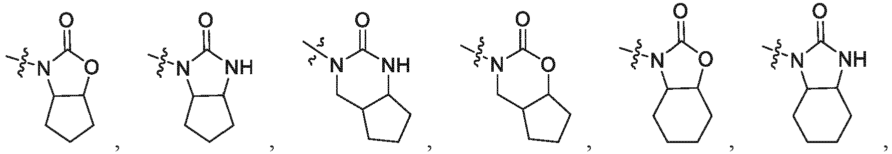 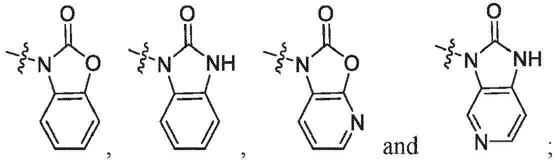 further, the ring Cy is optionally substituted with one or more R^{A3}.

Typical compounds of formula I include, but are not limited to: 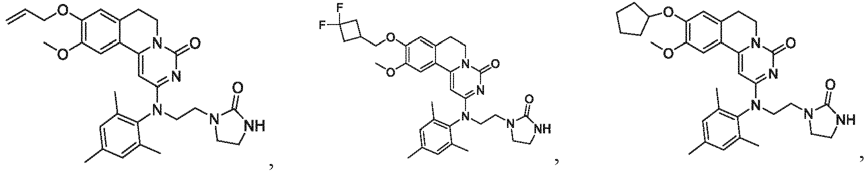 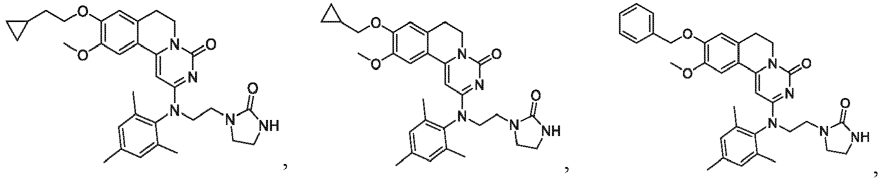 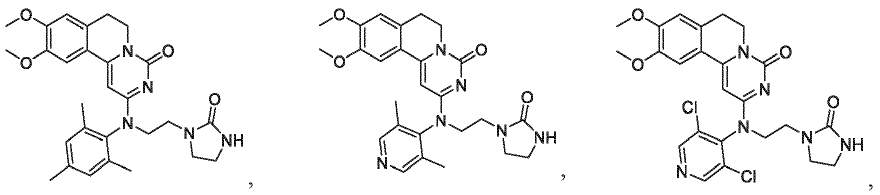 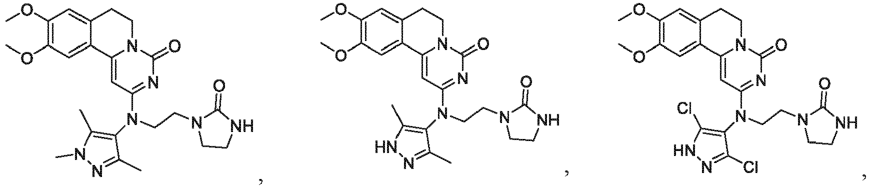 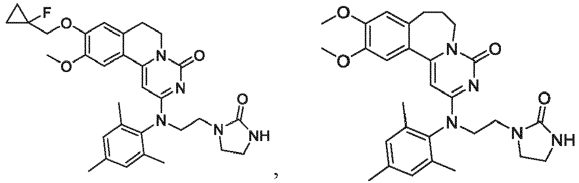 and

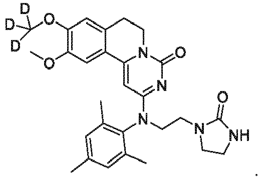

The disclosure further provides a pharmaceutical composition comprising a therapeutically effective amount of at least one of the compounds of formula I, II, III, IV or V or the pharmaceutically acceptable salts thereof described above and a pharmaceutically acceptable excipient.

In some embodiments, a unit dose of the pharmaceutical composition is 0.001 mg-1000 mg.

In certain embodiments, the pharmaceutical composition comprises 0.01-99.99% of the compound of formula I, II, III, IV or V or the pharmaceutically acceptable salt thereof described above based on the total weight of the composition. In certain embodiments, the pharmaceutical composition comprises 0.1-99.9% of the compound of formula I, II, III, IV or V or the pharmaceutically acceptable salt thereof described above. In certain embodiments, the pharmaceutical composition comprises 0.5%-99.5% of the compound of formula I, II, III, IV or V or the pharmaceutically acceptable salt thereof described above. In certain embodiments, the pharmaceutical composition comprises 1%-99% of the compound of formula I, II or III or the pharmaceutically acceptable salt thereof described above. In certain embodiments, the pharmaceutical composition comprises 2%-98% of the compound of formula I, II, III, IV or V or the pharmaceutically acceptable salt thereof described above.

The disclosure further provides a method for preventing and/or treating a PDE-related disorder comprising administering to a patient in need thereof a therapeutically effective amount of the compound of formula I, II, III, IV or V or the pharmaceutically acceptable salt thereof described above or the pharmaceutical composition described above.

In some embodiments, the PDE-related disorder is preferably asthma, obstructive pulmonary disease, sepsis, nephritis, diabetes, allergic rhinitis, allergic conjunctivitis, ulcerative enteritis or rheumatism.

The disclosure further provides a method for preventing and/or treating asthma, obstructive pulmonary disease, sepsis, nephritis, diabetes, allergic rhinitis, allergic conjunctivitis, ulcerative enteritis or rheumatism, comprising administering to a patient in need thereof a therapeutically effective amount of the compound of formula I, II, III, IV or V or the pharmaceutically acceptable salt thereof described above or the pharmaceutical composition described above.

The disclosure further provides use of the compound of formula I, II, III, IV or V or the pharmaceutically acceptable salt thereof described above or the pharmaceutical composition described above in the preparation of a medicament for preventing and/or treating a PDE-related disorder. In some embodiments, the PDE-related disorder is preferably asthma, obstructive pulmonary disease, sepsis, nephritis, diabetes, allergic rhinitis, allergic conjunctivitis, ulcerative enteritis or rheumatism.

The disclosure further provides use of the compound of formula I, II, III, IV or V or the pharmaceutically acceptable salt thereof described above or the pharmaceutical composition described above in the preparation of a medicament for preventing and/or treating asthma, obstructive pulmonary disease, sepsis, nephritis, diabetes, allergic rhinitis, allergic conjunctivitis, ulcerative enteritis or rheumatism.

In another aspect, the pharmaceutically acceptable salt of the compound in the disclosure is an inorganic or organic salt.

In another aspect, the compounds of the disclosure may exist in specific geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof are included within the scope of the disclosure.

The compounds and intermediates of the disclosure may also exist in different tautomeric forms, and all such forms are included within the scope of the disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

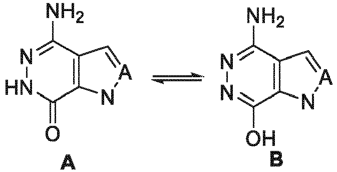

All the compounds in the present invention can be drawn as form A or form B. All tautomeric forms fall within the scope of the present invention. The nomenclature of the compounds does not exclude any tautomers.

The compounds of the disclosure may be asymmetric; for example, the compounds have one or more stereoisomers. Unless otherwise specified, all stereoisomers include, for example, enantiomers and diastereomers. The compounds of the disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers, and D- and L-isomers can be prepared by chiral synthesis, chiral reagents or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

The disclosure further includes isotopically-labeled compounds which are identical to those recited herein but have one or more atoms replaced by an atom having an atomic mass or mass number different from the atomic mass or mass number usually found in nature. Examples of isotopes that can be incorporated into the compound of the disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure further includes various deuterated forms of the compound of formula (I). Each available hydrogen atom connected to a carbon atom may be independently replaced by a deuterium atom. Those skilled in the art are able to synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

In the chemical structure of the compound of the disclosure, a bond " 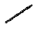 " represents an unspecified configuration; that is, if chiral isomers exist in the chemical structure, the bond " 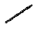 " may be " 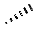 " or " " 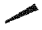 ", or contains both the configurations of " 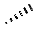 " and " 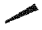 ". Although all of the above structural formulae are drawn as certain isomeric forms for the sake of simplicity, the disclosure may include all isomers, such as tautomers, rotamers, geometric isomers, diastereomers, racemates and enantiomers. In the chemical structure of the compound described herein, a bond " 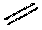 " is not specified with a configuration; that is, it may be in a Z configuration or an E configuration, or contains both configurations.

### Terms and definitions:

"Pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

"Pharmaceutically acceptable excipient" includes, but is not limited to, any auxiliary, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration as acceptable for use in humans or livestock animals.

"Effective amount" or "therapeutically effective amount" described herein includes an amount sufficient to ameliorate or prevent a symptom or disorder of a medical disorder. An effective amount also refers to an amount sufficient to allow or facilitate diagnosis. The effective amount for a particular patient or veterinary subject may vary with factors such as the disorder to be treated, the general health of the patient, the method and route and dosage of administration, and the severity of side effects. An effective amount may be the maximum dose or administration regimen to avoid significant side effects or toxic effects.

"Alkyl" refers to a saturated aliphatic hydrocarbon group, including linear and branched groups of 1 to 20 carbon atoms, and alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, and the like. Alkyl may be substituted or unsubstituted, and when it is substituted, the substituent may be substituted at any available point of attachment and is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

The term "cycloalkyl" refers to a saturated or partially unsaturated monocyclic or polycyclic hydrocarbon substituent. The cycloalkyl ring contains 3 to 20 carbon atoms, preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spiro cycloalkyl, fused cycloalkyl and bridged cycloalkyl.

The cycloalkyl ring may be fused to an aryl, heteroaryl or heterocycloalkyl ring, wherein the ring attached to the parent structure is cycloalkyl; non-limiting examples include indanyl, tetrahydronaphthyl, benzocycloheptyl, and the like. Cycloalkyl may be optionally substituted or unsubstituted, and when it is substituted, the substituent is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

The term "heterocycloalkyl" refers to a saturated or partially unsaturated monocyclic or polycyclic hydrocarbon substituent containing 3 to 20 ring atoms, one or more of which are heteroatoms selected from the group consisting of nitrogen, oxygen and S(O)ₘ (where m is an integer of 0 to 2), excluding a ring moiety of -O-O-, -O-S- or -S-S-, and the remaining ring atoms are carbon atoms. It preferably contains 3 to 12 ring atoms, 1 to 4 of which are heteroatoms; more preferably, it contains 3 to 7 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. Polycyclic heterocycloalkyl includes spiro heterocyclyl, fused heterocyclyl and bridged heterocycloalkyl. Non-limiting examples of "heterocycloalkyl" include:

| | | | | | |
|---|---|---|---|---|---|
| 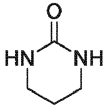 | 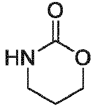 | 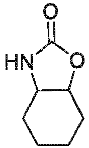 | 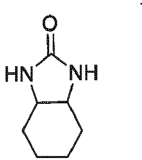 | 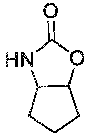 | 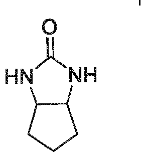 |
| 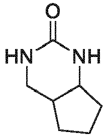 | 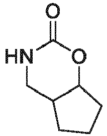 | 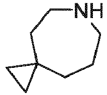 | 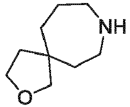 | 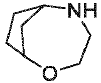 | 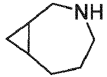 |
| 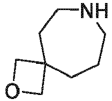 | 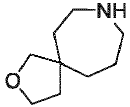 | 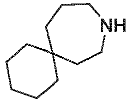 | 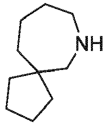 | 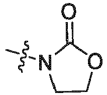 | 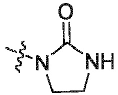 |

The heterocycloalkyl ring may be fused to an aryl, heteroaryl or cycloalkyl ring, wherein the ring attached to the parent structure is heterocycloalkyl; its non-limiting examples include:

| | | | | | |
|---|---|---|---|---|---|
| 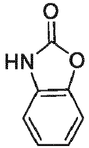 | 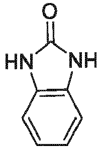 | 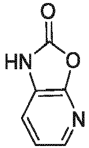 | 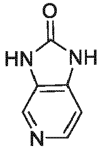 | 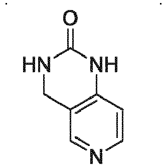 | 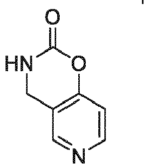 |
| 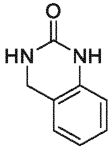 | 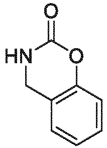 | 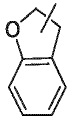 | 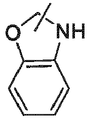 | 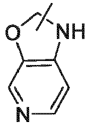 | 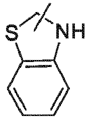 |

Heterocycloalkyl may be optionally substituted or unsubstituted, and when it is substituted, the substituent is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

"Alkenyl" refers to an unsaturated aliphatic linear or branched hydrocarbon group containing one or more carbon-carbon double bonds. Exemplary alkenyl groups include C2-C8, C2-C7, C2-C6, C2-C4, C3-C12 and C3-C6 alkenyl groups, including but not limited to, vinyl (i.e., vinyl), 1-propenyl, 2-propenyl (i.e., allyl), 2-methyl-1-propenyl, 1-butenyl, 2-butenyl (i.e., crotyl), and the like. Alkenyl used in any context herein is optionally substituted in the same manner as alkyl. Alkenyl used in any context herein may also be optionally substituted with aryl.

The term "aryl" refers to a 6- to 14-membered, preferably 6- to 12-membered, and more preferably 6- to 10-membered, all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocycloalkyl or cycloalkyl ring, wherein the ring attached to the parent structure is the aryl ring; its non-limiting examples include: 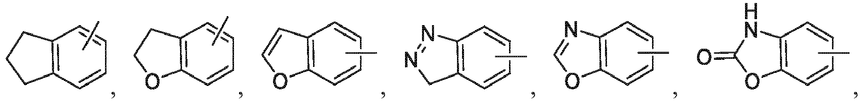 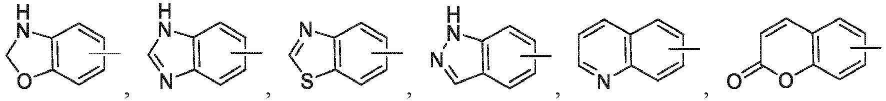 and

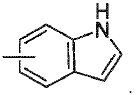

Aryl may be substituted or unsubstituted, and when it is substituted, the substituent is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

The term "heteroaryl" refers to a heteroaromatic system containing 1 to 4 heteroatoms and 5 to 14 ring atoms, wherein the heteroatoms are selected from the group consisting of oxygen, sulfur and nitrogen. Heteroaryl is preferably 5- to 12-membered, and is more preferably 5- or 6-membered. For example, its non-limiting examples include: imidazolyl, furyl, thienyl, thiazolyl, pyrazolyl, oxazolyl, pyrrolyl, tetrazolyl, pyridinyl, pyrimidinyl, thiadiazole, pyrazine, 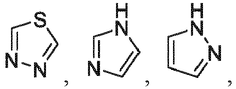 and the like.

The heteroaryl ring may be fused to an aryl, heterocycloalkyl or cycloalkyl ring, wherein the ring attached to the parent structure is heteroaryl; its non-limiting examples include:

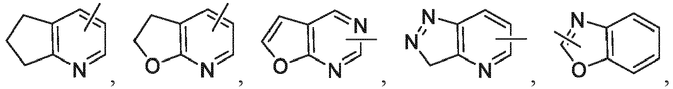

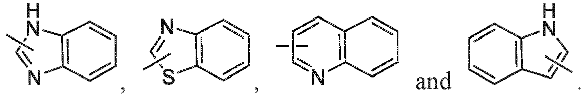

Heteroaryl may be optionally substituted or unsubstituted, and when it is substituted, the substituent is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy and cyclohexyloxy. Alkoxy may be optionally substituted or unsubstituted, and when it is substituted, the substituent is preferably one or more groups independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, aryl and heteroaryl are each independently optionally substituted with one or more substituents selected from the group consisting of deuterium, halogen, hydroxy, oxo, nitro, cyano and amino.

The term "alkenyloxy" refers to -O-alkenyl, wherein the alkenyl is as defined above.

The term "hydroxy" refers to the -OH group.

The term "halogen" refers to fluorine, chlorine, bromine or iodine.

The term "amino" refers to -NH₂.

The term "cyano" refers to -CN.

The term "nitro" refers to -NO₂.

The term "oxo" refers to the =O substituent.

"Optional" or "optionally" means that the event or circumstance subsequently described may, but does not necessarily, occur and that the description includes instances where the event or circumstance occurs or does not occur. For example, "a heterocycloalkyl group optionally substituted with alkyl" means that alkyl may, but does not necessarily, exist and that the description includes instances where the heterocycloalkyl group is or is not substituted with alkyl.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy group having free hydrogen is bound to a carbon atom having an unsaturated (e.g., olefinic) bond.

### BRIEF DESCRIPTION OF THE DRAWINGS

FIG. 1: a projection of the three-dimensional molecular structure of compound 1.
FIG. 2: the packing of single-crystal unit cells of compound 1.
FIG. 3: a projection of the three-dimensional molecular structure of compound 2.
FIG. 4: the packing of single-crystal unit cells of compound 2.

### DETAILED DESCRIPTION

The disclosure is further described below using examples. However, these examples do not limit the scope of the disclosure.

Experimental procedures without conditions specified in the examples of the disclosure were generally conducted according to conventional conditions, or according to conditions recommended by the manufacturers of the starting materials or commercial products. Reagents without specific origins indicated were commercially available conventional reagents.

The structures of compounds were determined by nuclear magnetic resonance (NMR) spectroscopy and/or mass spectrometry (MS). NMR shifts (δ) are given in 10⁻⁶ (ppm). NMR analysis was performed on a Bruker AVANCE-400 nuclear magnetic resonance instrument, with deuterated dimethyl sulfoxide (DMSO-*d₆*), deuterated chloroform (CDCl₃) and deuterated methanol (methanol-*d₄*) as solvents and tetramethylsilane (TMS) as an internal standard.

HPLC analysis was performed on a Waters 2795 AllianceHT LC high pressure liquid chromatograph with a Waters 2996 Photodiode Array Detector and a Thermo Accucore Polar Premium C18 50 × 4.6 mm 2.6 µm column.

MS analysis was performed on a Waters Micromass Quattro micro API triple quadrupole mass spectrometer in positive/negative ion scan mode, with the mass scan range being 120-1300.

X-ray single-crystal diffraction analysis was performed on a D8 Venture X-ray single-crystal diffractometer using a Mo target light source, a Mo-Ka (=0.71073 Å) X-ray, a CMOS plane detector, a 0.80 Å resolution, a 1.4 mA current, a 50 kV voltage, a 10 s exposure time, a 40 mm distance between the plane detector and the sample, and a 170 K test temperature.

Yantai Huanghai HSGF254 silica gel plates of 0.2 mm ± 0.03 mm layer thickness were adopted for thin-layer chromatography (TLC) analysis and 0.4 mm-0.5 mm layer thickness for TLC separation and purification.

Flash column purification was performed on a Combiflash Rf150 (TELEDYNE ISCO) or Isolara one (Biotage) system.

Normal phase column chromatography generally used 200-300 mesh or 300-400 mesh Yantai Huanghai silica gel as a carrier, or used a Changzhou Santai pre-fill ultrapure normal phase silica gel column (40-63 µm, 60 g, 24 g, 40 g, 120 g or other specifications).

Known starting materials in the disclosure may be synthesized using or according to methods known in the art, or may be purchased from Shanghai Titan Scientific, ABCR GmbH & Co. KG, Acros Organics, Aldrich Chemical Company, Accela ChemBio Inc., and Bide Pharmatech, among others.

In the examples, reactions can all be performed in a nitrogen atmosphere unless otherwise specified.

The nitrogen atmosphere means that the reaction flask is connected to a balloon containing about 1 L of nitrogen.

The hydrogen atmosphere means that the reaction flask is connected to a balloon containing about 1 L of hydrogen.

The hydrogen was prepared by a QPH-1L hydrogen generator from Shanghai Quan Pu Scientific Instruments Inc.

The nitrogen atmosphere or the hydrogenation atmosphere was generally formed by 3 cycles of vacuumization and nitrogen or hydrogen filling.

In the examples, a solution was an aqueous solution unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent for reactions, the eluent system for column chromatography purification of compounds, the developing solvent system for thin-layer chromatography and the volume ratio of the solvents were adjusted according to the polarity of the compound, or by adding a small amount of basic or acidic reagents such as triethylamine and acetic acid.

### Example 1

### Preparation of 9,10-dimethoxy-2-[[2-(2-oxo-imidazolin-1-yl)-ethyl]-(2,4,6-trimethyl-phenyl)-amino]-6 ,7-dihydro-pyrimido[6,1-a]isoquinolin-4-one (Compound 1

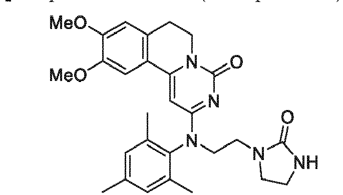

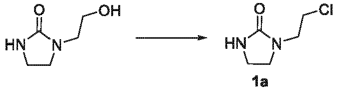

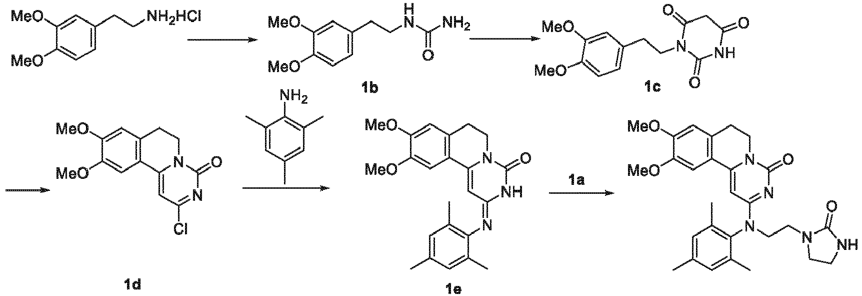

### Preparation of intermediate 1a: 1-(2-chloroethyl)-imidazolin-2-one

To 1-(2-hydroxyethyl)imidazolidinone (3.5 g, 26.9 mmol) at 0 °C was added slowly thionyl chloride (5 mL). The mixture was heated to 45 °C and stirred until the reaction was complete. A saturated solution of sodium chloride was added to quench the reaction, and the pH was adjusted to 7 with a 10% solution of NaOH. The mixture was extracted with dichloromethane, washed with a saturated solution of sodium chloride and dried over anhydrous sodium sulfate. The filtrate was concentrated under reduced pressure to give intermediate 1a (3.5 g, 88.4% yield), MS(ESI) m/z 149.1[M+H]⁺.

### Preparation of intermediate 1b: 1-(3,4-dimethoxyphenethyl)urea

2-(3,4-Dimethoxyphenyl)ethylamine hydrochloride (4.3 g, 19.8 mmol) was dissolved in water (25 mL) at room temperature, and the temperature was raised to 50 °C. Potassium cyanate (1.8 g, 21.8 mmol) was added portionwise, and stirring was continued until the reaction was complete. The mixture was cooled to 0 °C and filtered. The filter cake was washed with ice-cold water and dried to give intermediate 1b (4.1 g, 93.8% yield), MS(ESI) m/z 225.1 [M+H]⁺.

### Preparation of intermediate 1c: 1-[2-(3,4-dimethoxy-phenyl)-ethyl]-pyrimidin-2,4,6-trione

To absolute ethanol (50 mL) in an ice bath was added portionwise sodium ethoxide (3.8 g, 55.8 mmol). After the addition, the mixture was heated at reflux, and diethyl malonate (5.9 g, 36.6 mmol) was added dropwise. After the addition, the mixture was stirred for 0.25 h-0.5 h. A solution of intermediate 1b (4.1 g, 18.3 mmol) in ethanol (30 mL) was added dropwise. The mixture was stirred until the reaction was complete. The reaction mixture was cooled to 0 °C, and a 5% solution of HCl was added dropwise until the pH was 6. Water (300 mL) was added, and the mixture was filtered. The filter cake was washed with ice-cold water and dried to give intermediate 1c (3.9 g, 77.1% yield), MS(ESI) m/z 293.1 [M+H]⁺.

### Preparation of intermediate 1d: 2-chloro-9,10-dimethoxy-6,7-dihydropyrimido[6,1-a]isoquinolin-4-one

To phosphorus oxychloride (120 mL) at room temperature was added intermediate 1c (3.9 g, 13.4 mmol). The mixture was heated to 110 °C and stirred until the reaction was complete. The reaction mixture was cooled and concentrated, and the solid was poured into ice-cold water. A saturated solution of NaOH was added dropwise until the pH was 10, and the mixture was filtered. The filter cake was washed with ice-cold water and dried to give intermediate 1d (2.4 g, 62.4% yield), MS(ESI) m/z 293.1[M+H]⁺. Preparation of intermediate 1e: 9,10-dimethoxy-2-(2,4,6-trimethyl-phenylimino)-2,3,6,7-tetrahydropyrimido[6,1-a]isoq uinolin-4-one

Intermediate 1d (2.4 g, 8.2 mmol) was suspended in isopropanol (30 mL) at room temperature, and 2,4,6-trimethylaniline (4.5 g, 24.6 mmol) was added. The system was heated to 90 °C and stirred until the reaction was complete. The reaction mixture was cooled and filtered. The filter cake was washed with ice-cold water and dried to give intermediate 1e (3.0 g, 92.1% yield), MS(ESI) m/z 392.2[M+H]⁺.

### Preparation of compound 1: 9,10-dimethoxy-2-[[2-(2-oxo-imidazolin-1-yl)-ethyl]-(2,4,6-trimethyl-phenyl)-amino]-6 ,7-dihydro-pyrimido[6,1-a]isoquinolin-4-one

Intermediate 1e (0.72 g, 1.8 mmol) was dissolved in tetrahydrofuran (20 mL) at room temperature, and potassium tert-butoxide (0.42 g, 3.6 mmol) was added in a nitrogen atmosphere. After the addition, the mixture was heated to 65 °C, stirred for 48 h and cooled to 25 °C, and intermediate 1a (0.82 g, 5.5 mmol) was added. After the addition, the mixture was heated to 80 °C and stirred until the reaction was complete. The reaction mixture was quenched with a saturated solution of sodium chloride, extracted with dichloromethane, washed with a saturated solution of sodium chloride, and dried over anhydrous sodium sulfate. The filtrate was concentrated under reduced pressure and purified by silica gel column chromatography (n-heptane/ethyl acetate) to give the target compound 1 (0.21 g, 46.5% yield).

¹H NMR (400 MHz, CDCl₃) *δ* 6.99 (s, 2H), 6.69 (s, 1H), 6.64 (s, 1H), 5.39 (s, 1H), 4.61 (s, 1H), 4.22-4.15 (m, 2H), 4.08-3.99 (m, 2H), 3.93 (s, 3H), 3.77-3.69 (m, 5H), 3.55-3.46 (m, 2H), 3.38-3.42 (t, J = 6.8 Hz, 2H), 2.88-2.92 (t, J = 6.4 Hz, 2H), 2.34 (s, 3H), 2.18 (s, 6H).

MS(ESI) m/z 504.4[M+H]⁺.

Compound 1 was dissolved in 1 mL of 7% water/ethanol. The solution was filtered, and the filtrate was allowed to slowly evaporate at room temperature to give a rod-shaped crystal. Diffraction intensity data were collected using a D8 Venture X-ray single-crystal diffractometer.

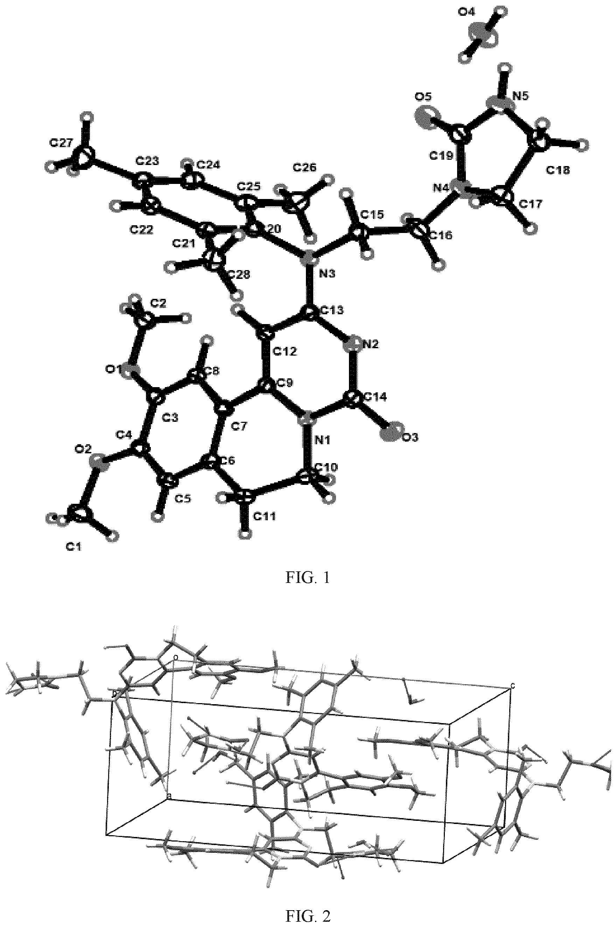

FIG. 1 shows a projection of the three-dimensional molecular structure. FIG. 2 shows the packing of single-crystal unit cells of the molecule.

### Example 2

### 10-Methoxy-9-(methoxy-d3)-2-[[2-(2-oxo-imidazolin-1-yl)-ethyl]-(2,4,6-trimethyl-phe nyl)-amino]-6,7-dihydro-pyrimido[6,1-a]isoquinolin-4-one (Compound 2)

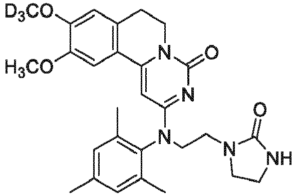

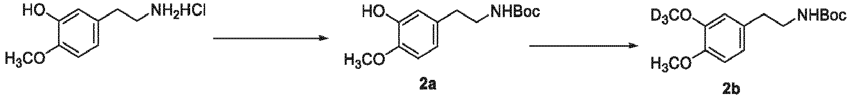

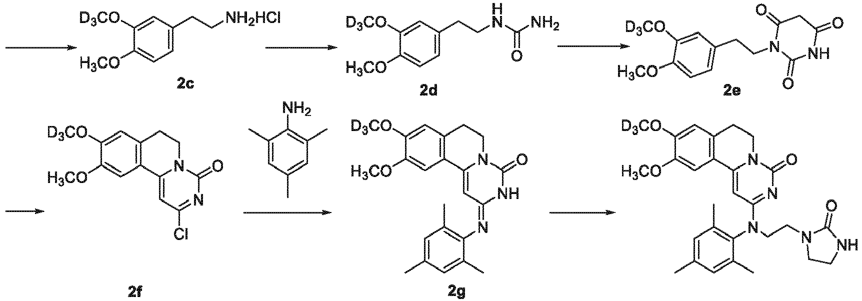

### Preparation of intermediate 2a: tert-butyl (3-hydroxy-4-methoxyphenethyl)carbamate

5-(2-Aminoethyl)-2-methoxyphenol hydrochloride (10.0 g, 49.2 mmol) was suspended in dichloromethane (120 mL), and triethylamine (13.4 g, 132.2 mmol) and (Boc)₂O (13.85 g, 63.5 mmol) were slowly added dropwise under ice-bath conditions. The mixture was slowly warmed to room temperature and stirred until the reaction was complete. The reaction mixture was quenched with a saturated solution of sodium chloride, extracted with dichloromethane, washed with a saturated solution of sodium chloride, and dried over anhydrous sodium sulfate. The filtrate was concentrated under reduced pressure to give the target compound 2a (10.6 g, 80.9% yield), MS(ESI) m/z 268.1[M+H]⁺.

### Preparation of intermediate 2b: tert-butyl (4-methoxy-3-(methoxy-d3)phenethyl)carbamate

Intermediate 2a (10.6 g, 39.7 mmol) and potassium carbonate (27.4 g, 198.0 mmol) were added to DMF (200 mL) at room temperature, and deuterated iodomethane (17.3 g, 119.1 mmol) was slowly added dropwise under ice-bath conditions. The mixture was slowly warmed to room temperature and stirred until the reaction was complete. The reaction mixture was quenched with a saturated solution of sodium chloride, extracted with ethyl acetate, washed with a saturated solution of sodium chloride, and dried over anhydrous sodium sulfate. The filtrate was concentrated under reduced pressure to give the target compound 2b (11.1 g, 99% yield), MS(ESI) m/z 285.2[M+H]⁺.

### Preparation of intermediate 2c: 2-(4-methoxy-3-(methoxy-d3)phenyl)ethan-1-amine hydrochloride

To intermediate 2b (11.1 g, 39.1 mmol) in ethyl acetate (20 mL) in an ice bath was added dropwise a 4 M solution of HCl/EA (150 mL). The mixture was slowly warmed to room temperature and stirred until the reaction was complete. The reaction mixture was filtered, and the filter cake was washed with ethyl acetate and dried to give intermediate 2c (7.1 g, 82.5% yield), MS(ESI) m/z 185.1[M+H]⁺.

### Preparation of intermediate 2d: 1-((4-methoxy-3-(methoxy-d3))phenethyl)urea

Intermediate 2c (7.1 g, 32.3 mmol) was dissolved in water (60 mL) at room temperature, and the temperature was raised to 50 °C. Potassium cyanate (4.7 g, 58.1 mmol) was added portionwise, and stirring was continued until the reaction was complete. The mixture was cooled to 0 °C and filtered. The filter cake was washed with ice-cold water and dried to give intermediate 2d (4.5 g, 61.6% yield), MS(ESI) m/z 228.2[M+H]⁺.

### Preparation of intermediate 2e: 1-((4-methoxy-3-(methoxy-d3))phenethyl)pyrimidine-2,4,6-trione

To absolute ethanol (60 mL) in an ice bath was added portionwise sodium ethoxide (4.1 g, 59.4 mmol). After the addition, the mixture was heated at reflux, and diethyl malonate (5.7 g, 35.6 mmol) was added dropwise. After the addition, the mixture was stirred for 0.25 h-0.5 h. A solution of intermediate 2d (4.5 g, 19.8 mmol) in ethanol (20 mL) was added dropwise. The mixture was stirred until the reaction was complete. The reaction mixture was cooled to 0 °C, and a 5% solution of HCl was added dropwise until the pH was 6. Water (300 mL) was added, and the mixture was filtered. The filter cake was washed with ice-cold water and dried to give intermediate 2e (4.3 g, 74.1% yield), MS(ESI) m/z 296.1[M+H]⁺.

### Preparation of intermediate 2f: 2-chloro-10-methoxy-9-(methoxy-d3)-6,7-dihydro-4H-pyrimido[6,1-a]isoquinolin-4-on e

To phosphorus oxychloride (63 mL) at room temperature was added intermediate 2e (4.3 g, 14.6 mmol). The mixture was heated to 110 °C and stirred until the reaction was complete. The reaction mixture was cooled and concentrated, and the solid was poured into ice-cold water. A saturated solution of NaOH was added dropwise until the pH was 10, and the mixture was filtered. The filter cake was washed with ice-cold water and dried to give intermediate 2f (4.2 g, 97.7% yield), MS(ESI) m/z 296.1[M+H]⁺.

### Preparation of intermediate 2g: 10-methoxy-9-(methoxy-d3)-2-((2,4,6-trimethyl-phenyl)-amino)-2,3,6,7-tetrahydro-4H-pyrimidinyl[6,1-a]isoquinolin-4-one

Intermediate 2f (4.2 g, 14.2 mmol) was suspended in isopropanol (40 mL) at room temperature, and 2,4,6-trimethylaniline (7.8 g, 42.6 mmol) was added. The mixture was heated to 90 °C and stirred until the reaction was complete. The reaction mixture was cooled and filtered. The filter cake was washed with isopropanol and dried to give intermediate 2g (5.1 g, 92.7% yield), MS(ESI) m/z 395.2[M+H]⁺.

### Preparation of compound 2: 10-methoxy-9-(methoxy-d3)-2-[[2-(2-oxo-imidazolin-1-yl)-ethyl]-(2,4,6-trimethyl-phen yl)-amino]-6,7-dihydro-pyrimido[6,1-a]isoquinolin-4-one

Intermediate 2g (0.5 g, 1.3 mmol) was dissolved in tetrahydrofuran (10 mL) at room temperature, and potassium tert-butoxide (0.31 g, 2.6 mmol) was added in a nitrogen atmosphere. After the addition, the mixture was heated to 65 °C and stirred for 48 h until the reaction was complete. The reaction mixture was quenched with a saturated solution of sodium chloride, extracted with dichloromethane, washed with a saturated solution of sodium chloride, and dried over anhydrous sodium sulfate. The filtrate was concentrated under reduced pressure and purified by silica gel column chromatography (n-heptane/ethyl acetate) to give the target compound 2 (0.07 g, 10.9% yield).

¹H NMR (400 MHz, CDCl₃) δ 6.96 (s, 2H), 6.68 (s, 1H), 6.64 (s, 1H), 5.36 (s, 1H), 4.31 (s, 1H), 4.15 (t, J = 6.4 Hz, 2H), 4.01 (t, J = 7.4 Hz, 2H), 3.70 (d, J = 7.6 Hz, 5H), 3.47 (t, J = 6.3 Hz, 2H), 3.39 (t, J = 7.9 Hz, 2H), 2.89 (t, J = 6.4 Hz, 2H), 2.31 (s, 3H), 2.15 (s, 6H).

MS(ESI) m/z 507.5[M+H]⁺.

Compound 2 was dissolved in 1 mL of ethanol. The solution was filtered, and the filtrate was allowed to slowly evaporate at room temperature to give an acicular crystal. Diffraction intensity data were collected using a D8 Venture X-ray single-crystal diffractometer.

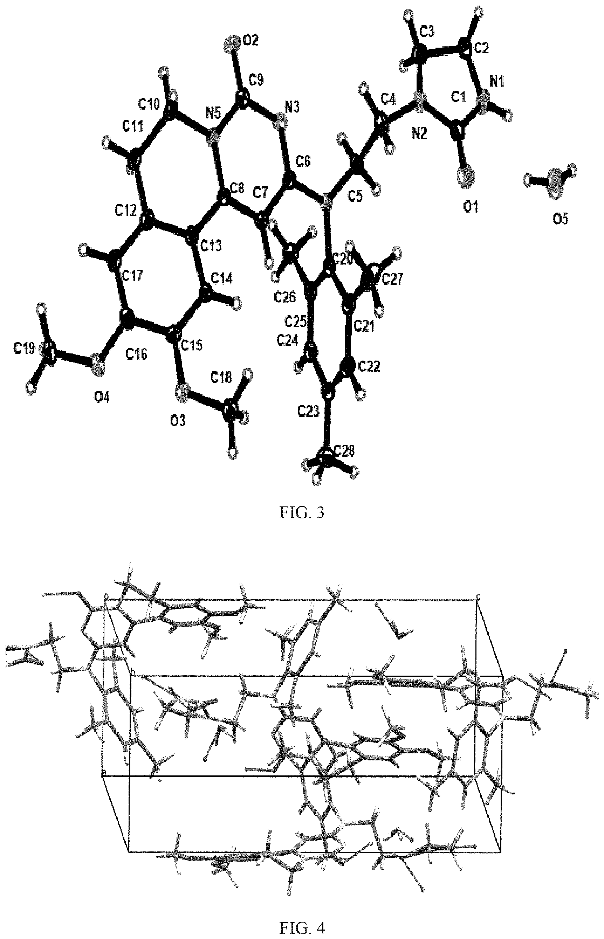

FIG. 3 shows a projection of the three-dimensional molecular structure. FIG. 4 shows the packing of single-crystal unit cells of the molecule.

### Comparative Example 1

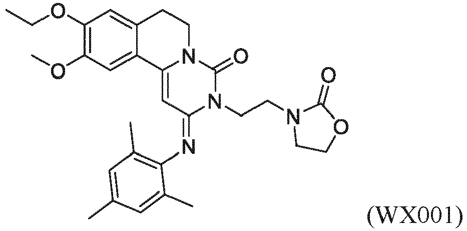

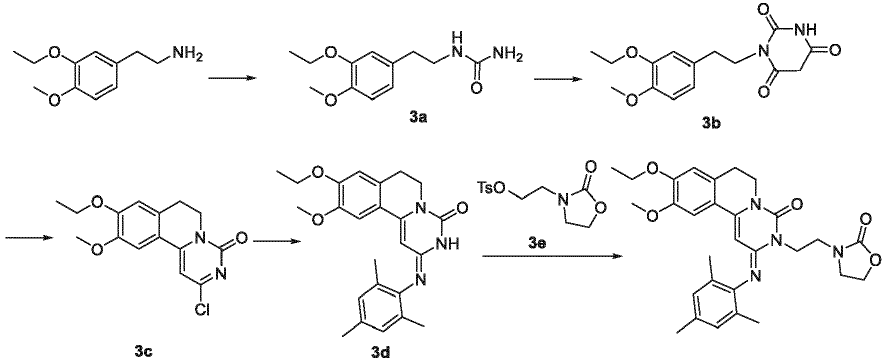

Intermediate 3d was prepared according to the method of example 1 using 2-(3-ethoxy-4-methoxyphenyl)ethylamine hydrochloride as a starting material. Intermediate 3d (1 g) was dissolved in 1,2-dichloroethane (20 mL) at room temperature, and 2-(2-oxooxazolidin-3-yl)ethyl 4-methylbenzenesulfonate (844 mg), potassium carbonate (612 mg) and sodium iodide (443 mg) were sequentially added. The mixture was heated to 80 °C and stirred until the reaction was complete. The reaction mixture was cooled, filtered, concentrated, diluted with water, and extracted with ethyl acetate. The organic phases were combined, dried, filtered, concentrated, and purified by column chromatography to give compound WX001. MS(ESI) m/z 519.0[M+H]⁺.

### Biological Evaluation

The disclosure is further described and explained below using test examples. However, these test examples are not intended to limit the scope of the disclosure.

### Test Example 1: In Vitro PDE4B Enzyme Activity Assay: An IMAP FP-Based Analysis Method

### 1. Materials

| Material | Brand | Catalog number/model |
|---|---|---|
| PDE4B1 | BPS | 60041 |
| Trequinsin | Sigma | T2057 |
| 384-well plate | Perkin Elmer | 6007279 |
| IMAP FP IPP assay kit | MOLECULAR DEVICES | R8124 |

### 2. Procedures

Compounds were serially diluted 5-fold with DMSO to different concentrations (10,000 nM, 2000 nM, 400 nM, 80 nM, 16 nM, 3.2 nM, 0.64 nM, 0.128 nM, 0.0256 nM and 0.005 nM). 200 µL of each of the compounds in different concentrations was added to a 384-well plate (n = 2), and meanwhile, 2 parts of 200 µL of DMSO were added to the 384-well plate (n = 2) as blank controls. Then 10 µL of a 0.025 µg/mL PDE4B1 enzyme solution (prepared with 1 mM 5× IMAP reaction buffer and 1 mM DTT) was added to the 384-well plate, and 10 µL of PDE4B1 enzyme-free blank buffer was added to one of the blank controls. The plate was incubated with shaking at room temperature for 15 min, and then 10 µL of a 0.1 µM FAM-cAMP solution (prepared with 1 mM 5× IMAP reaction buffer and 1 mM DTT) was added thereto. After the plate was incubated with shaking at room temperature for 30 min, 60 µL of an assay solution (prepared with 0.5625 mM 5× IMAP progressive binding buffer A, 0.1875 mM 5× IMAP progressive binding buffer B and 0.75 mM beads) was added. After the plate was incubated with shaking at room temperature for 60 min, data were collected. The formula for calculating inhibition rates is: inhibition rate = M/(M - M_{control}) × 100; IC₅₀ values were calculated from concentration-inhibition rate curves fitted. RPL554 was used as a positive control in this assay.

*In vitro* inhibition of PDE4B1 enzyme activity by the examples of the disclosure was measured through the above assay, and the inhibition rates and IC₅₀ values determined are shown in Table 1 and Table 2.

### Test Example 2: In Vitro PDE3A Enzyme Activity Assay: An IMAP FP-Based Analysis Method

### 1. Materials

| Material | Brand | Catalog number/model |
|---|---|---|
| PDE3A | BPS | 60030 |
| Trequinsin | Sigma | T2057 |
| 384-well plate | Perkin Elmer | 6007279 |
| IMAP FP IPP assay kit | MOLECULAR DEVICES | R8124 |

### 2. Procedures

Compounds were serially diluted 5-fold with DMSO to different concentrations (10,000 nM, 2000 nM, 400 nM, 80 nM, 16 nM, 3.2 nM, 0.64 nM, 0.128 nM, 0.0256 nM and 0.005 nM). 200 µL of each of the compounds in different concentrations was added to a 384-well plate (n = 2), and meanwhile, 2 parts of 200 µL of DMSO were added to the 384-well plate (n = 2) as blank controls. Then 10 µL of a 0.025 µg/mL PDE4B1 enzyme solution (prepared with 1 mM 5× IMAP reaction buffer and 1 mM DTT) was added to the 384-well plate, and 10 µL of PDE3A enzyme-free blank buffer was added to one of the blank controls. The plate was incubated with shaking at room temperature for 15 min, and then 10 µL of a 0.1 µM FAM-cAMP solution (prepared with 1 mM 5× IMAP reaction buffer and 1 mM DTT) was added thereto. After the plate was incubated with shaking at room temperature for 30 min, 60 µL of an assay solution (prepared with 0.5625 mM 5× IMAP progressive binding buffer A, 0.1875 mM 5× IMAP progressive binding buffer B and 0.75 mM beads) was added. After the plate was incubated with shaking at room temperature for 60 min, data were collected. The formula for calculating inhibition rates is: inhibition rate = M/(M - M_{control}) × 100; IC₅₀ values were calculated from concentration-inhibition rate curves fitted. RPL554 was used as a positive control in this assay.

*In vitro* inhibition of PDE3A enzyme activity by the examples of the disclosure was measured through the above assay, and the inhibition rates and IC₅₀ values determined are shown in Table 1 and Table 2.

**Table 1**

| No. | Inhibition rate (%) | |
|---|---|---|
| | PDE3A (500 nM) | PDE4B1(2000 nM) |
| RPL554 | 99.2 | 64.2 |
| Compound 1 | 100.3 | 82.8 |
| WX001 | 98.6 | 66.3 |

**Table 2**

| No. | IC₅₀ (nM) | |
|---|---|---|
| | PDE3A | PDE4B1 |
| RPL554 | 0.25 | 110 |
| Compound 1 | 0.13 | 50 |
| Compound 2 | 0.18 | 54 |
| WX001 | 0.90 | 876 |

| | | |
|---|---|---|
| Note: N/A stands for undetected. | | |

Conclusion: Compared to the positive compound RPL554, compounds 1 and 2 showed good biological activity in the *in vitro* enzyme assay, and compound 1 has 7 times higher inhibitory activity against PDE3A ezyme than the compound WX001, having good development prospects.

### Test Example 3: Pharmacokinetic (PK) Assay Using Intratracheal Administration

### 1. Objective

To evaluate the pharmacokinetic characteristics of test samples in SD rats and their distributions in lung tissue after intratracheal administration.

### 2. Test protocol

### 2.1. Test compounds

Compound 1, compound 2 and RPL-554

### 2.2. Test animals

198 ICR mice (Shanghai Slac Laboratory Animal Co., Ltd.), half being male and half female.

### 2.3. Compound preparation

### 1) Homogeneous solution:

0.5 g of tween 80 was weighed out and dissolved in 50 mL of a pH 2.5 citric acid/disodium hydrogen phosphate buffered salt solution for later use.

1.0 mg of test compound was weighed out and dissolved in a proper amount of the tween solution to prepare a 0.03 mg/mL for later use.

### 2) Suspension:

0.5 g of CMC-Na and 0.5 g of tween 20 were weighed out and well mixed with 50 mL of 0.9% normal saline solution to obtain a 1% CMC-Na and tween 20 solution for later use.

1.0 mg of test compound was weighed out, added to 10 mL of the above solution, dispersed ultrasonically, and well stirred to obtain a suspension for later use.

### 2.4. Administration regimens

| | Regimen 1 (suspension) | Regimen 2 (homogeneous solution) |
|---|---|---|
| Concentration | 0.15 mg/mL | 0.03 mg/mL |
| Volume | 40µL | 40µL |
| Dose | 6 µg/mouse | 1.2 µg/mouse |

### 3. Procedures/process

### 3.1. Intratracheal administration to mice

Mice were anesthetized with isoflurane gas and then administered compounds via the trachea. Time points for plasma collection were: 0.25, 0.5, 1, 2, 4, 8, 12 and 24 h. 200 µL of whole blood was collected, then anticoagulated with EDTA-K2, and centrifuged at 2-8 °C at a rate of about 6800 g for 6 min. The resulting plasma was transferred to properly labeled test tubes within 1 h of blood collection/centrifugation and stored at -80°C. Time points for lung tissue collection were: 0.5, 2, 8 and 24 h. The collected tissue samples were transferred to properly labeled test tubes and stored at -80°C.

### 3.2. Plasma treatment and LC-MS/MS analysis

Plasma samples (30.0 µL) were added to 1.5 mL centrifuge tubes, and 150 µL of internal standard working solution was added. The mixtures were vortexed for 1 min and centrifuged for 5 min (13,000 rpm, 4 °C). The supernatants (70.0 µL) were added to a 96-well plate, and 70.0 µL of deionized water was added. The mixtures were well mixed and then 2.00-µL samples were injected for LC-MS/MS analysis.

### 3.3. Lung tissue treatment

A proper amount of lung tissue samples was accurately weighed out and placed into homogenate tubes, and a volume (5 times the tissue weight) of acetonitrile was added. The tissue and acetonitrile were mixed and homogenized by ultrasonication for 5 min. Lung tissue homogenate samples (20.0 µL) were taken, and 30.0 µL of internal standard working solution and 200 µL of acetonitrile were added. The mixtures were vortexed for 1 min and centrifuged for 10 min (4000 rpm, 4 °C). The supernatants (100 µL) were added to a 96-well plate, and 100 µL of deionized water was added. The mixtures were well mixed by shaking the plate (1000 rpm, RT), and 1.00-µL samples were injected for LC-MS/MS analysis.

### 4. Pharmacokinetic parameters

Compared to RPL-554, compound 1 and compound 2 showed relatively high exposure *in vivo* and relatively long retention in the lung. The related data are shown in Tables 3 and 4.

**Table 3: The PK and tissue distribution of the suspension formulation**

| | Plasma | | | | Lung | | | |
|---|---|---|---|---|---|---|---|---|
| | Tₘₐₓ (h) | Cₘₐₓ (ng/ml) | AUC_{0-∞} (h*ng/ml) | T_{1/2} (h) | Tₘₐₓ (h) | Cₘₐₓ (ng/ml) | AUC_{0-∞} (h*ng/ml) | T_{1/2} (h) |
| RPL-554 | 0.25 | 9.55 | 8.19 | 0.88 | 24 | 5090 | 75300 | / |
| Compound 1 | 0.25 | 21.9 | 27.9 | 39.9 | 0.5 | 10700 | 126000 | 12.6 |
| Compound 2 | 0.25 | 33.9 | 66.2 | 9.15 | 0.5 | 14900 | 135000 | 10.2 |

**Table 4: The PK and tissue distribution of the homogeneous solution formulation**

| | Plasma | | | | Lung | | |
|---|---|---|---|---|---|---|---|
| | Tₘₐₓ (h) | Cₘₐₓ (ng/ml) | AUC_{0-∞} (h*ng/ml) | T_{1/2} (h) | Tₘₐₓ (h) | Cₘₐₓ (ng/ml) | AUC_{0-∞} (h*ng/ml) |
| RPL-554 | 0.25 | 5.77 | 2.1 | / | 0.5 | 35.1 | 38.5 |
| Compound 1 | 0.25 | 11.9 | 5.54 | 0.377 | 0.5 | 220 | 2040 |
| Compound 2 | 0.25 | 13.1 | 6.47 | 0.351 | 0.5 | 36.8 | 9.2 |

## Claims

1. A compound of formula I or a pharmaceutically acceptable salt thereof, 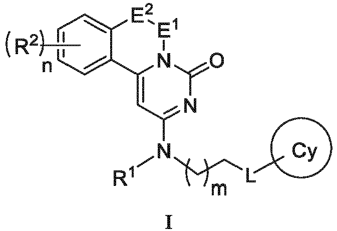
wherein R¹ is aryl or heteroaryl, and the aryl or heteroaryl is optionally substituted with one or more R^{A1};
each R^{A1} is independently selected from the group consisting of deuterium, halogen, hydroxy, nitro, amino, cyano, C₁₋₆ alkyl, C₁₋₆ alkoxy, C₃₋₇ cycloalkyl, C₃₋₇ cycloalkoxy, 3- to 7-membered heterocycloalkyl, 3- to 7-membered heterocycloalkoxy, C₆₋₁₀ aryl and 5- to 10-membered heteroaryl, and the alkyl, alkoxy, cycloalkyl, cycloalkoxy, heterocycloalkyl, heterocycloalkoxy, aryl and heteroaryl are each independently optionally substituted with one or more substituents selected from the group consisting of deuterium, halogen, hydroxy, oxo, nitro, cyano and amino;
each R² is independently selected from the group consisting of deuterium, halogen, hydroxy, nitro, amino, cyano, C₁₋₆ alkyl, C₂₋₇ alkenyl, C₃₋₇ cycloalkyl, 3- to 7-membered heterocycloalkyl, C₁₋₆ alkoxy, C₂₋₇ alkenyloxy, C₃₋₇ cycloalkoxy, 3- to 7-membered heterocycloalkoxy, C₆₋₁₀ aryl and 5- to 10-membered heteroaryl, and the hydroxy, amino, alkyl, alkenyl, cycloalkyl, heterocycloalkyl, alkoxy, alkenyloxy, cycloalkoxy, heterocycloalkoxy, aryl and heteroaryl are optionally substituted with one or more R^{A2};
each R^{A2} is independently selected from the group consisting of deuterium, halogen, hydroxy, nitro, amino, cyano, C₁₋₆ alkyl, C₁₋₆ alkoxy, C₃₋₇ cycloalkyl, 3- to 7-membered heterocycloalkyl, C₃₋₇ cycloalkoxy, 3- to 7-membered heterocycloalkoxy, C₆₋₁₀ aryl and 5- to 10-membered heteroaryl, and the alkyl, alkoxy, cycloalkyl, heterocycloalkyl, cycloalkoxy, heterocycloalkoxy, aryl and heteroaryl are each independently optionally substituted with one or more substituents selected from the group consisting of deuterium, halogen, hydroxy, oxo, nitro, cyano and amino;
E¹ is -(CH₂)_{q}-;
q is selected from the group consisting of 1, 2, 3 and 4;
E² is selected from the group consisting of -O-, -NH-, -S- and a single bond;
m is selected from the group consisting of 0, 1, 2, 3 and 4;
n is selected from the group consisting of 0, 1, 2, 3 and 4;
L is selected from the group consisting of -N(R⁶)-, -N(R⁶)C(O)-, -C(O)N(R⁶)-, -S-, -OC(O)-, -C(O)O-, -C(O)N(R⁶)CH₂-, -N(R⁶)C(O)CH₂-, -NHS(O)₂-, -S(O)₂NH- and a single bond;
R⁶ is selected from the group consisting of hydrogen, hydroxy and C₁₋₃ alkyl, and the alkyl is optionally substituted with one or more substituents selected from the group consisting of deuterium, halogen, hydroxy, oxo, nitro, amino and cyano;
ring Cy is heterocycloalkyl or heteroaryl, and the heterocycloalkyl or heteroaryl is optionally substituted with one or more R^{A3},
each R^{A3} is independently selected from the group consisting of deuterium, halogen, hydroxy, nitro, cyano, C₁₋₆ alkyl, C₃₋₇ cycloalkyl, 3- to 7-membered heterocycloalkyl, 6-to 10-membered aryl, 5- to 10-membered heteroaryl, SR', SOR', SO₂R', SO₂NR'(R"), NR'(R"), COOR' and CONR'(R"), and the alkyl, cycloalkyl, heterocycloalkyl, aryl, and heteroaryl are each independently optionally substituted with one or more substituents selected from the group consisting of deuterium, halogen, hydroxy, oxo, nitro, cyano and amino; and
R' and R" are each independently selected from the group consisting of hydrogen, deuterium, hydroxy, C₁₋₆ alkyl, C₃₋₇ cycloalkyl, 3- to 7-membered heterocycloalkyl, C₆₋₁₀ aryl and 5- to 10-membered heteroaryl, and the alkyl, cycloalkyl, heterocycloalkyl, aryl and heteroaryl are each independently optionally substituted with one or more substituents selected from the group consisting of deuterium, halogen, hydroxy, oxo, nitro, cyano and amino.

2. The compound or the pharmaceutically acceptable salt thereof according to claim 1, wherein ring Cy is 5- to 6-membered heterocycloalkyl, and the heterocycloalkyl is optionally substituted with one or more R^{A3}, and R^{A3} is as defined in claim 1; preferably, ring Cy is selected from the group consisting of 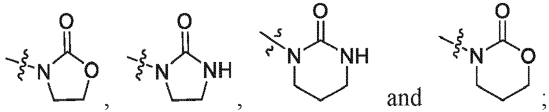 more preferably, ring Cy is 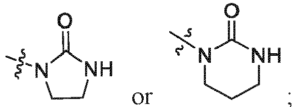 further, the ring Cy is optionally substituted with one or more R^{A3}, and R^{A3} is as defined in claim 1.

3. The compound or the pharmaceutically acceptable salt thereof according to claim 1 or 2, wherein E¹ is -(CH₂)q-, and q is 1 or 2; and E² is a single bond.

4. The compound or the pharmaceutically acceptable salt thereof according to any one of claims 1-3, wherein m is 1 or 2.

5. The compound or the pharmaceutically acceptable salt thereof according to any one of claims 1-4, wherein each R^{A3} is independently selected from the group consisting of deuterium, halogen, hydroxy, amino and C₁₋₆ alkyl, and the alkyl is optionally substituted with one or more substituents selected from the group consisting of deuterium, halogen, hydroxy, oxo, nitro, cyano and amino.

6. The compound or the pharmaceutically acceptable salt thereof according to any one of claims 1-5, being a compound of formula II or a pharmaceutically acceptable salt thereof, 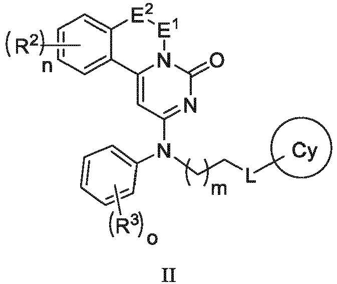
wherein each R³ is independently selected from the group consisting of deuterium, halogen, hydroxy, nitro, amino, cyano, C₁₋₆ alkyl, C₃₋₇ cycloalkyl, 3- to 7-membered heterocycloalkyl, C₁₋₆ alkoxy, C₃₋₇ cycloalkoxy, 3- to 7-membered heterocycloalkoxy, C₆₋₁₀ aryl and 5- to 10-membered heteroaryl, and the hydroxy, amino, alkyl, alkenyl, cycloalkyl, heterocycloalkyl, alkoxy, alkenyloxy, cycloalkoxy, heterocycloalkoxy, C₆₋₁₀ aryl and 5- to 10-membered heteroaryl are each independently optionally substituted with one or more substituents selected from the group consisting of deuterium, halogen, hydroxy, oxo, nitro, cyano and amino;
o is selected from the group consisting of 0, 1, 2, 3 and 4, preferably 0, 2 and 3; and ring Cy, R², m, n, L, E¹ and E² are as defined in claim 1.

7. The compound or the pharmaceutically acceptable salt thereof according to claim 6, wherein each R³ is independently selected from the group consisting of deuterium, halogen, hydroxy, amino, cyano, C₁₋₆ alkyl and C₁₋₆ alkoxy, and the alkyl is optionally substituted with one or more substituents selected from the group consisting of deuterium, halogen, hydroxy, oxo, nitro, cyano and amino; or each R³ is independently selected from the group consisting of C₃₋₇ cycloalkyl, C₃₋₇ cycloalkoxy, 3- to 7-membered heterocycloalkyl and 3- to 7-membered heterocycloalkoxy, and the cycloalkyl, cycloalkoxy, heterocycloalkyl and heterocycloalkoxy are each independently optionally substituted with one or more substituents selected from the group consisting of deuterium, halogen, hydroxy, oxo, nitro, cyano and amino.

8. The compound or the pharmaceutically acceptable salt thereof according to any one of claims 1-7, wherein each R² is independently selected from the group consisting of deuterium, halogen, hydroxy, amino and cyano; or each R² is independently selected from the group consisting of C₁₋₆ alkoxy, C₂₋₇ alkenyloxy, C₃₋₇ cycloalkoxy and 3- to 7-membered heterocycloalkoxy, and the alkoxy, alkenyloxy, cycloalkoxy and heterocycloalkoxy are each independently optionally substituted with one or more R^{A2}, and R^{A2} is as defined in claim 1.

9. The compound or the pharmaceutically acceptable salt thereof according to any one of claims 1-8, wherein L is selected from the group consisting of -N(R⁶)-, -N(R⁶)C(O)-, -C(O)N(R⁶)-, -NHS(O)z- and a single bond; or L is selected from the group consisting of -N(R⁶)C(O)-, -C(O)N(R⁶)- and a single bond, and R⁶ is as defined in claim 1.

10. The compound or the pharmaceutically acceptable salt thereof according to any one of claims 1-4 or 6-9, being a compound of formula IV or a pharmaceutically acceptable salt thereof, 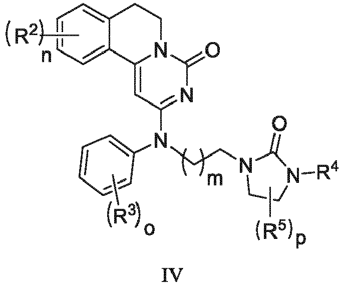
wherein R⁴ is selected from the group consisting of hydrogen, C₁₋₆ alkyl and C₃₋₇ cycloalkyl, and the alkyl and cycloalkyl are each independently optionally substituted with one or more substituents selected from the group consisting of deuterium, halogen, hydroxy, oxo, nitro, cyano and amino;
each R⁵ is independently selected from the group consisting of deuterium, halogen, hydroxy, nitro, cyano, C₁₋₆ alkyl, C₃₋₇ cycloalkyl, 3- to 7-membered heterocycloalkyl, 6-to 10-membered aryl and 5- to 10-membered heteroaryl, and the alkyl, cycloalkyl, heterocycloalkyl, aryl and heteroaryl are each independently optionally substituted with one or more substituents selected from the group consisting of deuterium, halogen, hydroxy, oxo, nitro, cyano and amino;
p is selected from the group consisting of 0, 1, 2, 3 and 4;
R², m and n are as defined in claim 1, and
R³ and o are as defined in claim 6.

11. The compound or the pharmaceutically acceptable salt thereof according to claim 10, wherein R⁴ is hydrogen or C₁₋₆ alkyl, and the alkyl is optionally substituted with one or more substituents selected from the group consisting of halogen, hydroxy and amino; and each R⁵ is independently selected from the group consisting of deuterium, halogen, hydroxy, nitro and cyano.

12. The compound or the pharmaceutically acceptable salt thereof according to any one of claims 1-11, being a compound of formula V or a pharmaceutically acceptable salt thereof, 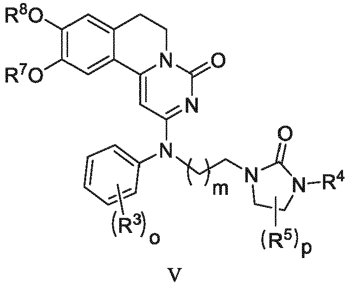
wherein R⁷ is selected from the group consisting of hydrogen, deuterium, C₁₋₆ alkyl, C₂₋₇ alkenyl, C₃₋₇ cycloalkyl and 3- to 7-membered heterocycloalkyl, and the alkyl, alkenyl, cycloalkyl and heterocycloalkyl are each independently optionally substituted with one or more R^{A4};
R^{A4} is selected from the group consisting of deuterium, halogen, hydroxy, oxo, nitro, amino, cyano, C₁₋₆ alkyl, C₁₋₆ alkoxy, C₃₋₇ cycloalkyl, 3- to 7-membered heterocycloalkyl, C₆₋₁₀ aryl and 5- to 10-membered heteroaryl, and the alkyl, alkoxy, cycloalkyl and heterocycloalkyl are each independently optionally substituted with one or more substituents selected from the group consisting of deuterium, halogen, hydroxy, oxo, nitro, cyano and amino;
R⁸ is selected from the group consisting of hydrogen, deuterium, C₁₋₆ alkyl, C₂₋₇ alkenyl, C₃₋₇ cycloalkyl and 3- to 7-membered heterocycloalkyl, and the alkyl, alkenyl, cycloalkyl and heterocycloalkyl are each independently optionally substituted with one or more R^{A5};
R^{A5} is selected from the group consisting of deuterium, halogen, hydroxy, oxo, nitro, amino, cyano, C₁₋₆ alkyl, C₁₋₆ alkoxy, C₃₋₇ cycloalkyl, 3- to 7-membered heterocycloalkyl, C₆₋₁₀ aryl and 5- to 10-membered heteroaryl, and the alkyl, alkoxy, cycloalkyl and heterocycloalkyl are each independently optionally substituted with one or more substituents selected from the group consisting of deuterium, halogen, hydroxy, oxo, nitro, cyano and amino;
m is as defined in claim 1;
R³ and o are as defined in claim 6; and
R⁴, R⁵ and p are as defined in claim 10.

13. The compound or the pharmaceutically acceptable salt thereof according to any one of claims 1-12, wherein the compound is selected from the group consisting of: 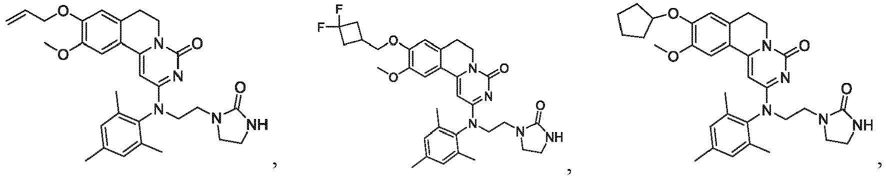 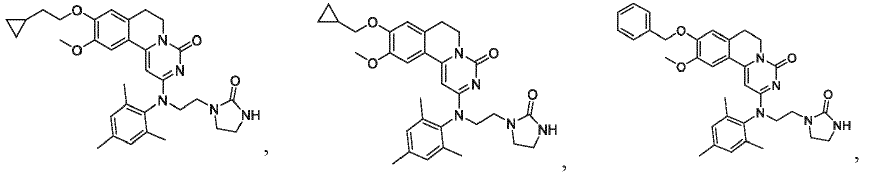 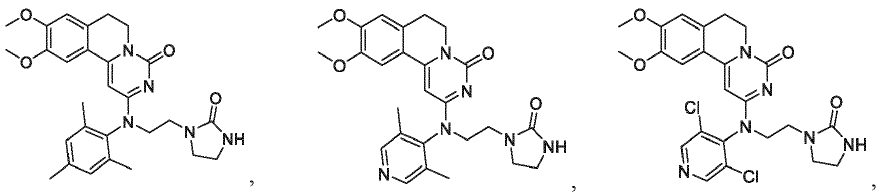 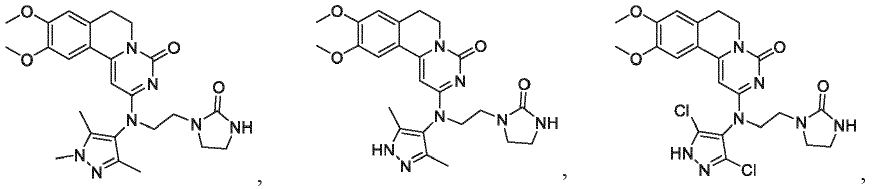 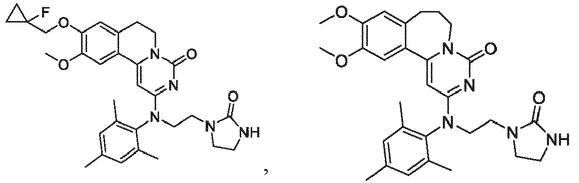 and

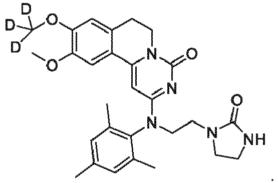

14. A pharmaceutical composition comprising a therapeutically effective amount of at least one of the compounds or the pharmaceutically acceptable salts thereof according to any one of claims 1-13 and a pharmaceutically acceptable excipient.

15. Use of the compound or the pharmaceutically acceptable salt thereof according to any one of claims 1-13 or the pharmaceutical composition according to claim 14 in the preparation of a medicament for preventing and/or treating a PDE-related disorder.

16. Use of the compound or the pharmaceutically acceptable salt thereof according to any one of claims 1-13 or the pharmaceutical composition according to claim 14 in the preparation of a medicament for preventing and/or treating asthma, obstructive pulmonary disease, sepsis, nephritis, diabetes, allergic rhinitis, allergic conjunctivitis, ulcerative enteritis or rheumatism.
